# EUROPÄISCHE PATENTSCHRIFT

(11) **EP 1 771 217 B1**
(45) Veröffentlichungstag und Bekanntmachung des Hinweises auf die Patenterteilung: **16.03.2011**
(21) Anmeldenummer: 05763584.9
(22) Anmeldetag: 14.07.2005
(51) Int. Cl.: A61L 27/50

(54) **FÜLLMITTEL UND ZUFÜHRVORRICHTUNG ZUM AUSBILDEN EINER STÜTZSTRUKTUR IN EINEM KNOCHENINNENRAUM**
FILLER AND ADMINISTRATION DEVICE FOR FORMING A SUPPORTIVE STRUCTURE IN A BONE CAVITY
MOYEN DE REMPLISSAGE ET DISPOSITIF D'INTRODUCTION POUR ÉTABLIR UNE STRUCTURE DE SOUTIEN DANS UNE CAVITE INTRAOSSEUSE

(30) Priorität: 14.07.2004 EP 04405451
(43) Veröffentlichungstag der Anmeldung: 11.04.2007
(73) Patentinhaber: Akross AG, 8200 Schaffhausen (CH)
(72) Erfinder: Sidler, Bruno, 5737 Menziken (CH)
(74) Vertreter: Graf, Werner
(86) Internationale Anmeldenummer: PCT/EP2005/007936
(87) Internationale Veröffentlichungsnummer: WO 2005/094735

(56) Entgegenhaltungen:
- WO-A-01/54746
- WO-A-2004/041075
- WO-A-2004/047689
- US-A- 5 676 700
- US-A1- 2003 055 511
- US-A1- 2004 052 829
- US-A1- 2004 097 930
- US-B1- 6 582 439
- CUNIN G ET AL: "EXPERIMENTAL VERTEBROPLASTY USING OSTEOCONDUCTIVE GRANULAR MATERIAL" SPINE, PHILADELPHIA, PA, US, Bd. 25, Nr. 9, 1. Mai 2000 (2000-05-01), Seiten 1070-1076, XP008007548

## Beschreibung

Die vorliegende Erfindung betrifft ein Füllmittel zum Ausbilden einer Stützstruktur in einem Knocheninnenraum gemäss dem Oberbegriff von Anspruch 1. Die Erfindung betrifft weiter eine Zufuhrvorrichtung zum Einfüllen eines Füllmittels in einen Knocheninnenraum gemäss dem Oberbegriff von Anspruch 22.

Die Erfindung bezieht sich auf die Behandlung von Knochen bei Menschen oder Tieren.

Die zunehmende Alterung der Gesellschaft führt zu einer überdurchschnittlichen Zunahme von Erkrankungen des Bewegungs- und Stützapparates, insbesondere der Knochen. Knochen können auf unterschiedlichste Arten geschädigt oder geschwächt werden, beispielsweise durch Trauma, Infektion, Abnutzung, Tumorwachstum oder regenerative Krankheiten wie Osteoporose. Bei älteren Menschen stellt insbesondere die Osteoporose, das heisst der Abbau der Spongiosa, ein Problem dar, weil dadurch die Belastungsfähigkeit des Knochens geschwächt wird, was zur Folge hat, dass vermehrt Knochenbrüche auftreten, vor allem an der Wirbelsäule, am Schenkelhals und am Handgelenk. Eine Behandlung derartiger Knochenbrüche ist schwierig, insbesondere wenn degenerative Veränderungen im fortgeschrittenen Stadium vorliegen. Zur Fixation derartiger Knochenbrüche werden üblicherweise äussere oder innere Schienungen (Platte, Schrauben, Implantate) verwendet, welche den Knochen bis zu dessen Heilung zusammenhalten. Derartige Schienen sind jedoch nicht bei allen Knochen verwendbar. So wird beispielsweise bei der Wirbelsäule zur Behandlung eines degenerierten oder eingebrochenen Wirbelkörpers die sogenannte Vertebroplastie angewandt, indem der geschädigte Wirbelkörper mit Knochenzement gefüllt wird. Die Verwendung von Knochenzement bei Wirbelkörpern weist jedoch verschiedene Nachteile auf, insbesondere dass der Knochenzement über Venen oder kleine Knochendefekto im Wirbelkörper unkontrolliert anstreten karm, und dadurch Schaden in den benachbarten anatomischen Strukturen verursachen kann, beispielsweise bei einem Austritt in den Spinalkanal. Weitere Nachteile von Knochenzement sind, dass sich dieser beim Aushärten stark erwärmt, was umliegendes Gewebe oder sogar Nerven schädigen kann, dass der Knochenzement sehr schnell verarbeitet werden muss, dass im Knochenzement kein Knochen nachwachsen kann, und dass der Knochenzement mit der Zeit spröde wird.

Die Druckschrift US 2004/0052829 offenbart ein Verfahren zur Behandlung poröser Wirbelkörper, insbesondere von Wirbelkörpern mit Osbeoporosefraktur. Dazu wird eine biokompatible, flüssige Trägersubstanz wie Wasser verwendet, welche mit biokompatibles Stützkörpern angereichert ist Nachteilig an diesem Verfahren ist die Tatsache, dass ein ballonartiger Behälter erforderlich ist, welcher zuerst in den Wirbelkörper einzubringen ist, und welcher danach mit der Trägersubstanz enthaltend die Stützkörper gefüllt wird. Der ballonartige Behälter ist erforderlich um sicherzustellen, dass sowohl die flüssige Trägersubstanz als auch die darin enthaltenen Stützkörper innerhalb des Wirbelkörpers verbleiben. Würde kein ballonartiger Behälter verwendet, so bestünde auch bei diesem Verfahren die bekannte Gefahr des Ausfliessens, indem die flüssige Trägersubstanz sowie die darin enthaltenen Stützkörper, welche eine Grösse im Mikometerbereich aufweisen, aus dem geschädigte Wirbelkörper entweichen, und auf unkkontrollierbare Art benachbartes Gewebe schädigen oder sich im menschlichen Körper ausbreiten. Das bekannte Verfahren weist somit die Nachteile auf, dass es aufwendig ist den ballonartigen Behälter in den Wirbelkörper einzubringen, dass dieser beim Einbringen oder durch die Stützkörper beschädigt werden kann, sodass die Trägersubstanz ausfliessen könnte, und dass der Wirbelkörper nicht optimal füllbar ist, da sich der ballonartige Behälter während dem Zuleiten der flüssigen Trägersubstanz "aufbläst", und somit mehr Volumen als für die Stützkörper an sich erforderlich beansprucht.

Die Druckschrift US 2004/0097930 offenbart ein weiteres Verfahren zur Behandlung von Wirbelkörpern. Dieses Verfahren offenbart kugelförmige Körper, welchen in den Innenraum des Wirbelkörpers eingeführt werden. Nach erfolgter Operation wird die Wirbelsäule in axialer Richtung, das heisst in Verlaufsrichtung der Wirbelsäule belastet. Diese Belastung hat zur Folge, dass die kugelförmigen Körper radial zur Wirbelsäulenachse verdrängt werden, wodurch der Wirbelkörper einsinkt und sich verformt. Im ungünstigsten Fall durchdringen die kugelförmigen Körper die Umhüllung des Wirbelkörpers, und verteilen sich danach unkontrolliert im menschlichen Körper. Die Druckschrift offenbart zudem viereckige Körper, welcher in den Innenraum des Wirbelkörpers eingeführt werden sollen. Diese viereckigen Körper blockieren sich während dem Zuführen gegenseitig derart, dass diese Körper dem Innenraum des Wirbelkörpers gar nicht zugeführt werden können, Das offenbarte Verfahren weist somit die Nachteile auf, dass die in den Wirbelkörpers eingeführten Körper unter Belastung entweichen möchten, und/oder dass die einzuführenden Körper dem Wirbelkörper nicht zugeführt werden können.

Es ist daher Aufgabe der vorliegenden Erfindung ein vorteilhafteres, implantierbares, insbesondere injizierbares Füllmittel vorzuschlagen, welches defekte Knochen, insbesondere Wirbelkörper, derart optimal zu versorgen erlaubt, dass die Knochen vom Zeitpunkt der Implantation an fähig sind die anliegenden physiologischen Lasten zu tragen.

Diese Aufgabe wird gelöst mit einem trockenfliessfähigen Füllmittel aufweisend die Merkmale von Anspruch 1, Die Unteransprüche 2 bis 21 betreffen weitere vorteilhaft ausgestaltete Füllmittel. Die Aufgabe wird weiter gelöst mit einer Zuführvorrichtung zum Einführung des Füllmittels aufweisend die Merkmale von Anspruch 22. Die Unteransprüche 22 und 26, betreffen weitere vorteilhafte Ausgestaltungen.

Die Aufgabe wird weiter gelöst mit einem System aufweisend die Merkmale von Anspruch 27.

Die Aufgabe wird insbesondere gelöst mit einem trockenfliessfähigen Füllmittel zum Ausbilden einer Stützstruktur in einem Knocheninneraum, wobei das Füllmittel eine Mehrzahl biokompatibler Stützkörper umfasst, welche unter den üblicherweise im Knocheninnenraum auftretenden physiologischen Lasten resistent gegen Verformung oder Bruch sind, wobei die Stützkörper eine Grösse zwischen 2 mm und 10 mm aufweisen, und wobei die Stützkörper eine Achse aufweisen, sowie zwei gegenüberliegende, in Richtung der Achse beabstandete Stossstellen. Die Stossstellen sind vorzugsweise als Stossflächen ausgestaltet

Der Ausdruck "trockenfliessfähig" bedeutet, dass das Füllmittel injizierbar ist, jedoch ohne Verwendung irgendeiner fluiden Trägersubstanz, welche den Stützkörpern Fliesseigenschaften verleihen könnte. Die Stützkörper sind derart gross gewählt, dass diese, zum Beispiel in einer Kanüle hintereinander liegend angeordnet, alle gemeinsam in der Kanüle verschiebbar sind, indem auf den hintersten Stützkörper ein Druck ausgeübt wird, und diese Kraft auf alle sich in der Kanüle befindlichen Stützkörper übertragen wird, sodass die Stützkörper in der Kanüle zur Kanülenspitze hin bewegt werden. Die Stützköper sind in der Kanüle derart ausgerichtet angeordnet, dass die Stossstellen zweier nacheinander folgender Stützkörper jeweils aneinander liegen. Diese Stützkörper weisen trockenfliessfähige Eigenschaften auf, indem diese in der Kanüle ohne eine Trägersubstanz eine Art fliessende Eigenschaften aufweisen, indem die Stützkörper, ähnlich einer Injektion, über die Kanüle einem Knocheninnenraum zuführbar sind.

Der Ausdruck "Stützkörper" bezeichnet einen Körper, welcher die in einem Knocheninnenraum, insbesondere die in einem Wirbelkörper auftretenden Kräfte zu tragen vermag, ohne wesentlich verformt oder gar zerstört zu werden. Es sind eine Vielzahl von biokompatiblen Werkstoffen bekannt, aus welchen ein derartiger Stützkörper gefertigt sein kann. Beispielsweise könnte der Stützkörper gefertigt sein aus:
- keramischen Werkstoffen, insbesondere Calciumphosphate / Hydroxylapatite, Aluminiumoxide, Zirkoniumoxide, ATZ Keramik (Alu-Zirkonium-Oxid), bioaktive Gläser, Glaskeramiken, Porzellan oder einer Kombination davon, oder
- metallischen Werkstoffen, insbesondere Titan, Tantal, rostfreier Stahl, StahIlegienmgen wie Kobalt-Chrom Legierung Titanlegierungen wie Titan-Nickel Legierung oder Titan-Aluminium-Niobium/Vanadium Legierung, oder einer Kombination davon, oder
- Polymeren, insbesondere Polymethylmethacryl (PMMA), Polyetheretherketon (PEEK) Polyethylen (PE), Polyethylenterephthalat (PET), oder einer Kombination davon, oder
- biodegradablen Polymeren wie Polylactate.

Das erfindungsgemässe Füllmittel umfassend eine Mehrzahl biokompatibler Stützkörper weist die Vorteile auf, dass kein Ballon oder ein anderes Begrenzungsmittel im Knocheninnenraum erforderlich ist, da das Füllmittel einerseits keine Trägersubstanz wie eine Flüssigkeit aufweist, welche auslaufen könnte, dass das Füllmittel in der Kanüle trockenfliessfähig ist, und dass das Füllmittel andererseits aus einzelnen, relativ grossen Stützkörpern besteht, welche auf Grund ihrer Grösse auch einem stark geschädigten Wirbelkörper kaum unkontrolliert entwichen können, und sich zudem auf Grund ihrer Grösse nicht unkontrolliert im Körper verteilen können. Das erfindungsgemässe Füllmittel ist trockenfliessfähig und kann daher mit Hilfe einer Kanüle in einen Knocheninnenraum injiziert werden. Das erfindungsgemässe Füllmittel ist daher insbesondere auch zur Versorgung schwer zugänglicher Knochen wie der Wirbelknochen geeignet.

In einer besonders vorteilhaften Ausführungsform sind die Stützkörper derart ausgestaltet, dass sie sich gegenseitig verkeilen können, sodass die sich im Knocheninnenraum befindlichen Stützkörper gegenseitig verkeilen und dadurch eine zusammenhängende Stützstruktur bilden. Vorzugsweise sind die Stützkörper derart verkeilt, dass diese eine eigenstabile Stützstruktur bilden. Dies ist insbesondere bei belasteter Wirbelsäule wichtig.

Das erfindungsgemässe Füllmittel erlaubt Knochendefekte wie frakturierte Knochen, insbesondere geschwächte, frakturierte oder eingefallene Wirbelkörper, mit einer Stützstruktur bestehend aus einzelnen Stützkörpern zu füllen. Zudem können eingefallene Wirbelkörper wieder aufgerichtet werden. Die Stützstruktur verleiht dem Knochen eine Stabilität, überträgt auftretende Kräfte, und kann zudem die Knochenheilung und/oder die Knochenbildung anregen. Die Stützkörper können zudem mit knochenheilenden und/oder knochenbildenden Substanzen gefüllt und/oder beschichtet sein oder eine knochenheilende und/oder knochenbildende Oberflächenstruktur aufweisen. Das erfindungsgemässe Füllmittel ist mittels einer Zuführvorrichtung umfassend eine kleine Kanüle in den Knochen einbringbar, was einen schonenden Zugang zum Knochen ermöglicht. In einer vorteilhaften Ausgestaltung umfasst die Zuführvorrichtung eine Kraftmessvorrichtung, um die während dem Einführen in Förderrichtung auf die Stützkörper bewirkte Kraft zu Messen. Dadurch wird während dem Einführen eine übermässige Kraft vermieden. Dadurch wird ein Durchbrechen der Aussenwand des Wirbelkörpers vermieden. Zudem steht dem Arzt über die Einführkraft ein Indikator zur Verfügung, welcher etwas über den Füllzustand des Wirbelkörpers aussagt. In einer weiteren vorteilhaften Ausgestaltung umfasst die Zuführvorrichtung eine Antriebsvorrichtung, welche einen in der Kanüle angeordneten Stössel betätigt. Die Antriebsvorrichtung übt auf den Stössel und die davor angeordneten Stützkörper in Zuführrichtung eine mechanische Kraft aus, beispielsweise eine konstante Kraft, einen Schlag oder eine Vibration. In einer weitern Ausgestaltung weist die Zuführvorrichtung einen bis in den Knocheninnenraum vorragenden Stössel auf, dessen Spitze als Manipulationsinstrument dient, um die Lage der sich innerhalb des Knocheninnenraumes befindlichen Stützkörper zu verändern, beziehungsweise um diese auszurichten.

Die Erfindung wird nachfolgend an Hand von Figuren im Detail beschrieben. Es zeigen:
Fig. 1 eine Aufsicht auf einen Wirbelkörper mit einer eingeführten Kanüle;
Fig. 2 einen Schnitt durch einen Wirbelkörper, in welchen das Füllmittel eingeführt wird;
Fig. 3 einen Schnitt durch den Wirbelkörper sowie die Kanüle gemäss Fig. 2;
Fig. 4 einen Schnitt durch den Wirbelkörper mit vollständig eingeführtem Füllmittel;
Fig. 5a bis 5e schematische Darstellungen unterschiedlich ausgebildeter Stützstrukturen;
Fig. 6a bis 6e Stützkörper mit eckigen Aussenkonturen;
Fig. 7a bis 7d Stützkörper mit abgerundeten Aussenkonturen;
Fig. 8a bis 8d Stützkörper mit polyederförmigen Aussenkonturen;
Fig. 9 ein weiteres Ausführungsbeispiel eines teilweise mit Füllmittel gefüllten Knochenhohlraumes;
Fig. 10 das Befestigen einer Kanüle in einem Knochen;
Fig. 11 eine Kanüle;
Fig. 12 eine Kanülenspitze;
Fig. 13 eine Pressvorrichtung;
Fig. 14 ein gebrochener Knochen mit Knochenhohlraum;
Fig. 15 ein Querschnitt durch eine Kanüle;
Fig. 16 ein weiterer Querschnitt durch eine Kanüle;
Fig. 17 einen Längsschnitt durch einen weiteren Wirbelkörper mit Zuführvorrichtung und einzuführenden Stützkörpern;
Fig. 18a-18e unterschiedlich ausgestaltete Stösselspitzen;
Fig. 19 einen Querschnitt durch einen weiteren Wirbelkörper mit eingeführten Stützkörpern sowie eine Draufsicht auf eine Zuführvorrichtung;
Fig. 20a-20c Längsschnitte unterschiedlich ausgestalteter Stützkörper;
Fig. 21 eine Umfangsansicht einer Stossstelle;
Fig. 22a-b drei einzelne, ineinander angeordnete Stützkörper;
Fig. 23 ein weiteres Ausführungsbeispiel eines Stützkörpers.

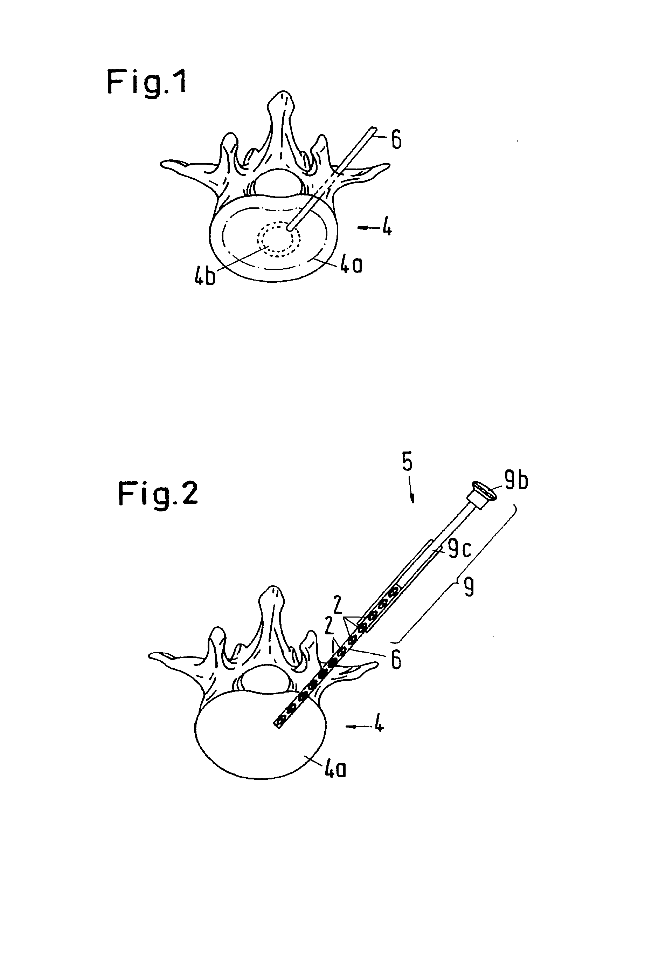

Figur 1 zeigt in einer Aufsicht einen Knochen 4, spezifischer einen Wirbelkörper mit einem Knocheninnenraum 4a. Unter dem Begriff Knocheninnenraum 4a wird das gesamte, vom Knochen 4 eingenommene Volumen verstanden. Im Knocheninnenraum 4a befindet sich ein Knochenhohlraum 4b, in welchen eine Kanüle 6 mündet. Der Knochenhohlraum 4b kann beispielsweise auf Grund degenerativer Vorgänge, insbesondere Osteoporose, entstanden sein. Der Knochenhohlraum 4b kann mit einem geeigneten Instrument auch künstlich erzeugt oder vergrössert werden.

Figur 2 zeigt einen Wirbelkörper 4 mit einem Knocheninnenraum 4a sowie eine Zuführvorrichtung 5 umfassend eine Kanüle 6, eine Pressvorrichtung 9 mit Betätigungsgriff 9b und einem Stössel 9c. Die Kanüle 6 münden in den Knocheninnenraum 4a, welcher in diesem Ausführungsbeispiel keinen Knochenhohlraum 4b aufweist. Im Innenraum der Kanüle 6 und der Pressvorrichtung 9 sind biokompatible Stützkörper 2 hintereinander liegend und sich im wesentlichen gegenseitig berührend angeordnet, sodass der auf den zuhinterst liegenden Stützkörper 2 wirkende Stössel 9c alle Stützkörper 2 in Richtung Knocheninnenraum 4a treibt. Dies ermöglicht, falls erforderlich, über die Stützkörper 2 die am Betätigungsgriff 9b anliegende Kraft bis zu den sich an der Spitze der Kanüle 6 befindlichen Stützkörpern 2 zu übertragen, sodass diese mit einer entsprechenden Kraft in den Knocheninnenraum 4a eindringen, und dabei während dem Eindringen einen Knochenhohlraum 4b ausbilden. Der Innendurchmesser der Kanüle 6 sowie der Pressvorrichtung 9 sind bezüglich dem Aussendurchmesser der Stützkörper 2 derart angepasst ausgestaltet, dass die Stützkörper 2 in Flussrichtung, d.h. zur Austrittsöffnung der Kanüle 6 hin, hintereinanderliegend angeordnet sind, was zur Folge hat, dass die Gesamtheit der Stützkörper 2 ein trockenfliessfähiges Füllmittel 1 bilden, welches ohne jegliche Schmiermittel fliessähnliche Eigenschaften aufweist, indem die Stützkörper 2 mit einer wie in Figur 2 dargestellt spritzenähnlichen Vorrichtung dem Knocheninnenraum 4a zuführbar ist. Die Kanüle 6 könnte auch gekrümmt verlaufen, oder aus einem flexiblen oder festen Material bestehen.

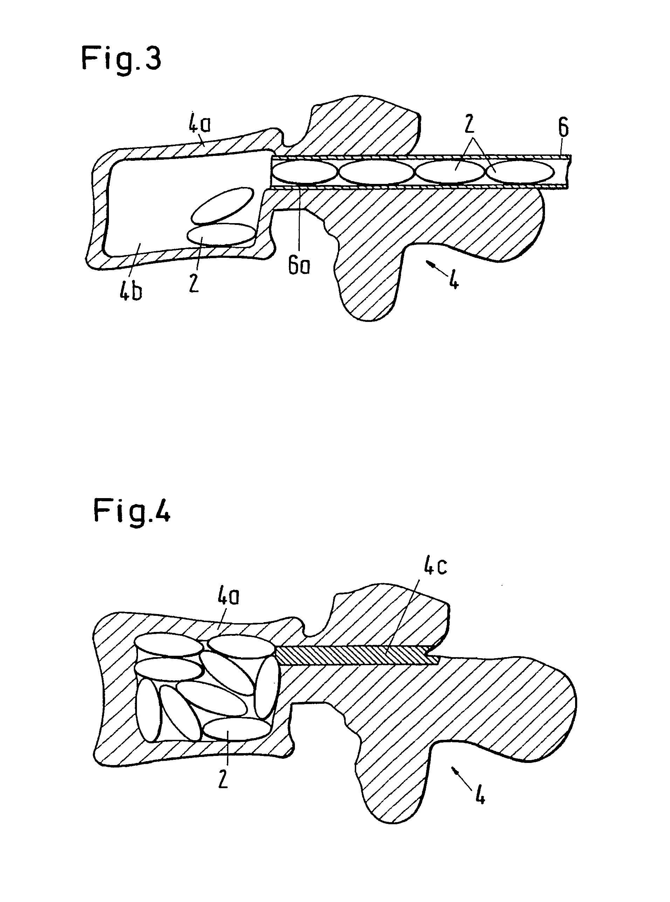

Der in Figur 3 in einem Schnitt dargestellte Wirbelkörper 4 weist einen Knochenhohlraum 4b auf, welcher vorgängig, vor dem Einführen der Stützkörper 2, mit einem speziellen Instrument erzeugt wurde. Danach wird die Spitze 6a der Kanüle 6 bis zum Knochenhohlraum 4b vorgetrieben, und danach die in einer Seitenansicht dargestellten Stützkörper 2 einzeln, nacheinander folgend, und in definierter gegenseitiger Lage zugeführt, sodass sich diese zufällig im Knochenhohlraum 4b verteilen. Die Kanüle 6 weist einen runden Innenquerschnitt auf, sodass jeder Stützkörper 2, welche alle identisch ausgestaltet sind, eine längliche, sphärisch verlaufende Aussenkontur aufweist. Der Stützkörper 2 weist eine maximale Dimension zwischen 2 mm und 10 mm auf. Die Stützkörper 2 sind als Vollkörper ausgestaltet. Alle Stützkörper 2 sind betreffend Dimension und Form identisch ausgestaltet.

Figur 4 zeigt den in Figur 3 dargestellten Wirbelkörper 4 mit einem vollständig durch Stützkörper 2 gefüllten Knochenhohlraum 4b, wobei die Stützkörper 2 an unterschiedlichen Stelle aufeinander aufliegen und dabei im Knochenhohlraum 4b gefangen sind, sodass die Gesamtheit dieser Stützkörper 2 eine tragfähige Stützstruktur bilden. Der Zugangskanal zum Knochenhohlraum 4b wurde nach dem Einführen der Stützkörper 2 mit einem Zapfen 4c verschlossen.

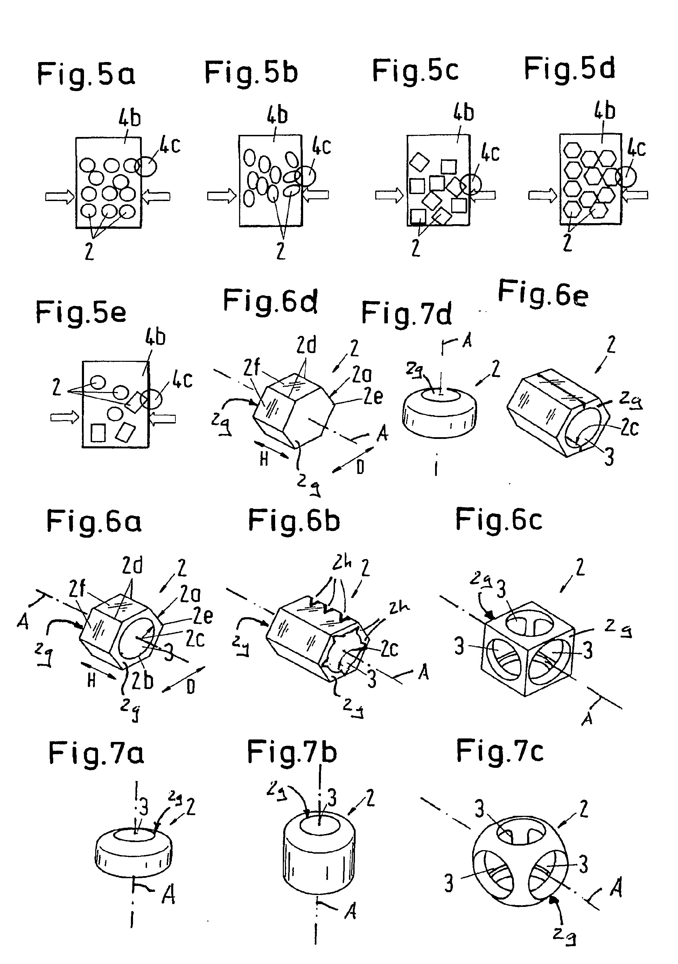

Ein Knocheninnenraum 4a beziehungsweise ein Knochenhohlraum 4b kann mit unterschiedlichst ausgestalteten Stützkörpern 2 gefüllt werden. Figur 5a zeigt schematisch einen begrenzten Knochenhohlraum 4b mit Einlassöffnung 4c, durch welche die kugelförmigen Stützkörper 2 eingeführt werden. Figur 5b zeigt ellipsoidförmig ausgestaltete Stützkörper 2. Ein Vorteil der in den Figuren 5a und 5b dargestellten Stützkörpern 2 mit sphärischer Aussenkontur ist die Tatsache, dass sie unter geringem Kraftaufwand gegenseitig verschiebbar sind, sodass diese Stützkörper 2 das Volumen des Knochenhohlraumes 4b sehr gut ausfüllen. Die Figuren 5c und 5d zeigen Stützkörper 2 mit einer kantigen Aussenkontur, wobei die unter einem Winkel zusammenlaufenden Seitenflächen der Stützkörper 2 eine Keilwirkung ausüben können, sodass sich die im begrenzten Knochenhohlraum 4b befindlichen Stützkörper 2 in einer vorteilhaften Ausführungsform, wie in Figur 5c dargestellt, gegenseitig verkeilen, wobei sich zwischen dem Stützkörper 2 grössere Zwischenräume ergeben, in welche der Knochen nachwachsen kann. Die Gesamtheit der Stützkörper 2 bildet wiederum eine mechanisch belastbare Stützstruktur. Im Gegensatz zu den in den Figuren 5a bis 5d dargestellten Stützkörpern 2, welche jeweils bezüglich ihrer Form und Grösse identisch sind, könnten die Stützkörper 2, wie in Figur 5e dargestellt, innerhalb des Knochenhohlraumes 4b auch unterschiedliche Formen und/oder Grössen aufweisen.

Die Figuren 6a-6d und 7a-7d zeigen Stützkörper 2, welche eine Achse A aufweisen, wobei jeder Stützkörper 2 zwei gegenüberliegende, in Richtung der Achse A beabstandete Stossstellen 2g aufweist. Die Stossstellen 2g der Figuren 6a-6e sowie der Figur 7d sind als Stossflächen 2g ausgestaltet, wogegen die Stossstellen 2g der Figuren 7a-7c als Ringe mit geringer gegenseitiger Berührungsfläche ausgestaltet sind. In den Ausführungsbeispielen gemäss der Figuren 6a-6e verlaufen die Stossstellen 2g senkrecht zur Achse A. In den Ausführungsbeispielen gemäss der Figuren 7a-7d weisen die Stossstellen 2g einen kreisförmigen oder sphärischen beziehungsweise bombierten Verlauf auf. Die Stossstellen 2g der Ausführungsbeispiele gemäss den Figuren 7a-7c könnten auch ringförmig, senkrecht zur Achse A verlaufend ausgestaltet sein. Die Stützkörper 2 weisen in den Figuren 6a-6c, 7a-7c eine zur Stossstelle 2g hin angeordnete Ausnehmung 2c auf. Diese Ausnehmung 2c kann als Vertiefung ausgestaltet sein, oder auch als ein durchgehender, offener Innenhohlraum 3, welche sich zwischen den beiden gegenüberliegenden Stossstellen 2g erstreckt, und in die gegenüberliegenden Ausnehmungen 2c mündet. Die Ausnehmung 2c kann exzentrisch, oder wie in den Figuren 6a-6c, 7a-7c dargestellt, konzentrisch zur Achse A verlaufen. Die Stützkörper 2 könnten auch, wie in Figur 6e dargestellt, aus zwei oder auch noch mehr Teilkörpern bestehen, beispielsweise aus vier Teilkörpern, welche sich im Knocheninnenraum voneinander unabhängig bewegen können.

Die Figuren 6a, 6b und 6c sowie 7a, 7b und 7c zeigen Stützkörper 2 mit einem offenen Innenhohlraum 3. Unter einem offenen Innenhohlraum 3 wird ein Hohlraum im Stützkörper 2 verstanden, welcher gegen Aussen offen ist, im Gegensatz zu einem geschlossenen Hohlraum, welcher vollständig im Innern des Stützkörpers 2 angeordnet ist, ohne eine Öffnung nach Aussen aufzuweisen. Auch diese Stützkörper 2 weisen eine maximale Grösse im Bereich zwischen 2 mm und 10 mm auf. Jeder Stützkörper 2 umfasst ein Gesamtvolumen, welches dem Volumen des Materials des Stützkörpers 2 sowie dessen Innenhohlraum 3 entspricht. Das Volumen des Innenhohlraumes ist grösser als 30% des Gesamtvolumens, vorzugsweise grösser als 50% und kann bis zu 90% betragen. Die Grösse des maximal möglichen Volumens des Innenhohlraumes 3 ist abhängig von den maximal am Stützkörper 2 angreifenden Druckkräften. Diese Druckkräfte sind abhängig vom spezifischen Knocheninnenraum 4a beziehungsweise von Knochen 4, in welchem die Stützstruktur gebildet wird. Die Tragfähigkeit des Stützkörpers 2 ist natürlich abhängig vom verwendeten Material. Wird der Stützkörper 2 beispielsweise aus Metall gebildet, so kann der Innenhohlraum 3 relativ gross ausgebildet sein, und der Stützkörper 2 trotzdem resistent gegen die angreifenden Druckkräfte sein. Ist der Stützkörper 2 aus einem Material wie Bioglas oder einer resorbierbaren Substanz gefertigt, so muss der Innenhohlraum 3 entsprechend den Materialeigenschaften prozentual kleiner ausgebildet sein, um dem Stützkörper 2 eine genügend grosse Tragkraft zu verleihen. Dieser Innenhohlraum 3, insbesondere ein prozentual relativ grosser Innenhohlraum 3, weist den Vorteil auf, dass dieser mit der Zeit von nachwachsendem Knochenmaterial gefüllt werden kann. In einer besonders vorteilhaften Ausgestaltung wird der Innenhohlraum 3 des Stützkörpers 2 vor dessen Einführen zumindest teilweise mit einer osteoinduktiven und/oder osteokonduktiven Substanz gefüllt, insbesondere einem knochenwachstumsfördernden Protein oder Kalziumsulfat oder einer Kombination dieser oder weiterer Substanzen. Dank derart gefüllten Stützkörpern 2 ist es möglich dem Knocheninnenraum 4a mittels dem trockenfliessfähigen Füllmitteln 1, bestehend aus einer Vielzahl von Stützkörpern 2, sowohl eine Stützstruktur als auch osteoinduktive und/oder osteokonduktive Substanzen zuzuführen. Diese Ausgestaltung weist die Vorteile auf, dass das Füllmittel 1 trocken dem Knocheninnenraum 4a zugeführt wird, sodass keine Gefahr eines Auslaufens durch eventuell im Knochen 4 vorhandene Ritzen, Spalten und Öffnungen besteht. Zudem bewirkt die osteoinduktive Substanz ein Knochenwachstum, sodass die Hohlräume 3 und die sich zwischen den Stützkörpern 2 ergebenden Zwischenräume vorteilhafterweise zunehmend mit nachwachsendem Knochen gefüllt werden. Da die Stützkörper 2 im Knocheninnenraum 4a zufällig ausgerichtet angeordnet sind, das heisst die Hohlräume 3 in zufälligen Richtungen verlaufen, und auch die Zwischenräume in zufälligen Richtungen verlaufen und eine durch den Zufall bestimmte Grösse aufweisen, bilden die Stützkörper 2 an sich, insbesondere wenn das Volumen des Innenhohlraumes 3 mehr als 50% des Gesamtvolumens beträgt, eine im weitesten Sinne Spongiosa ähnliche Stützstruktur. Wenn mit zunehmendem Heilungsverlauf zudem die Hohlräume 3 und die Zwischenräume mit nachwachsender Spongiosa gefüllt werden, so bildet sich im Knocheninnenraum 4a eine dem gesunden Knochen teilweise vergleichbare Stützstruktur aus, mit willkürlich ausgerichteten Stützkörpern 2, deren Hohl- und Zwischenräume mit Spongiosa verwachsen sind.

Der Innenhohlraum 3 der Stützkörper 2 gemäss der Figuren 6a,6b,6c,7a,7b,7c ist zusammenhängend und nicht porös ausgestaltet, sodass die Innenkontur 2b seitlich einen relativ grossen Innenhohlraum 3 begrenzt. Der Innenhohlraum 3 gemäss der Figuren 6a,6b,7a,7b ist zylinderförmig ausgestaltet, verläuft konzentrisch zur Achse A, und weist eine kreisförmige Öffnung 2c auf. Der Winkel zwischen der Innenwand des Innenhohlraumes 3 und de Stossstelle 2g beträgt an der kreisförmige Öffnung 2c somit 90 Grad. Die kreisförmige Öffnung 2c kann, wie in Figur 6b dargestellt, Blockierstellen 2h aufweise, beispielsweise eine Mehrzahl in Umfangsrichtung angeordneter Kerben. Die Öffnung 2c kann unterschiedlichste Formen aufweisen, und zum Beispiel auch als Dreieck, Viereck oder als Polygonzug ausgestaltet sein. Der Innenhohlraum 3 könnte dieselbe, durch die Öffnung 2c vorgegebenen Form aufweisen, wobei sich der Innenhohlraum 3 in Achsrichtung A über die gesamte Länge des Stützkörpers 3 erstrecken kann. Die Stützkörper 2 gemäss den Figuren 7a, 7b sind im Wesentlichen ringförmig ausgestaltet. Die Stützkörper 2 gemäss den Figuren 6a, 6b weisen in Umfangsrichtung eine polyederförmige Aussenkontur auf, mit parallel zur Achse A verlaufenden Kanten 2d, und sechs gegenseitig keilförmig verlaufenden Oberflächen 2f, welche sich jeweils in einer Kante 2d bzw. Ecke 2e treffen. Der Stützkörper 2 weist in Umfangsrichtung vorzugsweise eine 3- bis 10-eckige Aussenkontur auf, insbesondere eine 4-, 5- oder 6-eckige Aussenkontur. Die Kanten 2d können, wie in Figur 6b dargestellt, Blockierstellen 2h wie Kerben aufweisen.

Die Ausführungsform gemäss Figur 7a weist in Umfangsrichtung eine sphärisch, insbesondere kugelförmig oder ellipsoidförmig verlaufende Aussenkontur auf. Die Stützkörper 2 gemäss den Figuren 6b und 7b sind im Wesentlichen hohlzylinderförmig ausgestaltet. Die Stützkörper 2 gemäss den Figuren 6c und 7c weisen im Wesentlichen eine würfelförmige bzw. sphärisch verlaufende Aussenkontur auf. Die Stützkörper 2 gemäss den Figuren 6d und 7d weisen, im Unterschied zu den Ausführungsformen gemäss den Figuren 6a, 7a, keinen Hohlraum 3 auf.

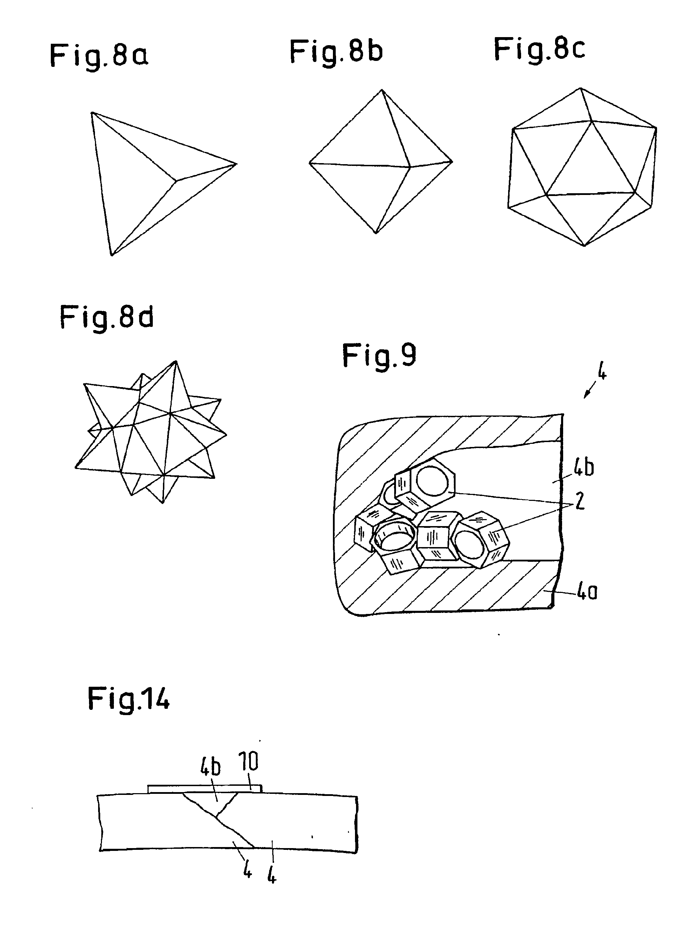

Die Figuren 8a bis 8d zeigen weitere Ausführungsform von Stützkörpern 2 mit polyederförmig verlaufenden Aussenkonturen auf, wobei die dargestellten Stützkörper 2 keinen Hohlraum 3 aufweisen. Die Stützkörper 2 könnten jedoch auch einen Hohlraum 3 aufweisen. Figur 8a zeigt ein Tetraeder, Figur 8b ein Oktaeder, Figur 8c ein Ikosaeder und Figur 8d ein kleines Sternendodekaeder. Nicht dargestellt sind in diesen Ausführungsformen die zwei gegenüberliegenden, in Richtung der Achse A beabstandeten Stossstellen 2g, welche erforderlich sind, um diese Ausführungsformen in der Kanüle in Richtung der Achse A zu fördern.

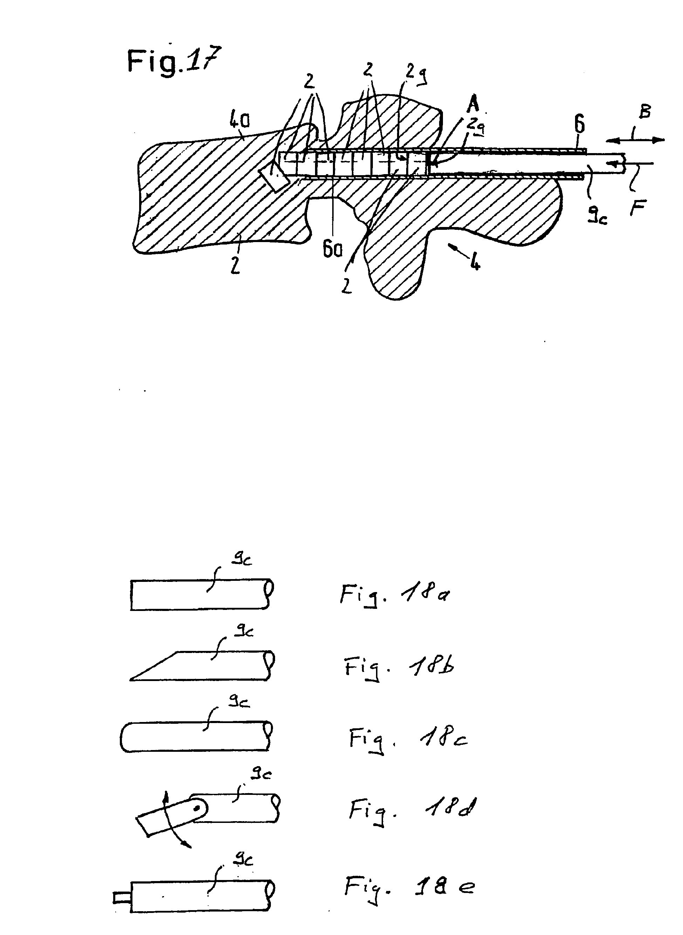

In einer vorteilhaften Ausgestaltung weist der Stützkörper 2, wie in Figur 6a dargestellt einen Aussendurchmesser D und eine dazu orthogonale, beziehungsweise in Richtung der Achse A verlaufende Höhe H auf, wobei der Aussendurchmesser D vorzugsweise zumindest das 1,5-fache der Höhe H beträgt. Die Stützkörper 2 weisen eine derart geformte Oberfläche auf, dass die Oberfläche eines in den Knocheninnenraum eingefüllten Stützkörpers 2 in die Ausnehmung eines benachbart angeordneten Stützkörpers 2 hineinragen kann, wie dies in Figur 9 dargestellt ist. Die Stützkörper 2 weisen in einer vorteilhaften Ausgestaltung derart ausgebildete Oberflächen sowie derart ausgestaltete Ausnehmungen auf, dass zwischen den in Ausnehmungen hineinragenden Oberflächen und der Ausnehmung ein gegenseitiges Verkeilen beziehungsweise Verklemmen auftritt, um innerhalb des Knocheninnenraumes 4a eine vorzugsweise zusammenhängende, und insbesondere eine eigenstabile Stützstruktur zu bilden. Die sich radial zur Achse A befindlichen Oberfläche der Stützkörper 2 weist daher vorzugsweise Formen aus der Gruppe Ecke, Kante, Spitze, Vertiefung, Durchbrechung oder eine Kombination davon auf, um ein gegenseitiges Verkeilen und Verklemmen der Stützkörper 2 zu bewirken. In einer vorteilhaften Ausgestaltung weist der Stützkörper 2, wie in Figur 6a dargestellt, eine Schraubenmutter ähnliche Form auf, mit einer in Verlaufsrichtung der Achse A mehreckigen Aussenkontur mit gegenseitig keilförmig verlaufenden Flächen 2f und markanten Kanten 2d und Ecken 2e, sowie einem Innenhohlraum 3 mit einer Öffnung 2c mit relativ grossem Durchmesser. Figur 9 zeigt einen erst teilweise mit derartigen, schraubenmutterförmigen Stützkörpern 2 gefüllten Knochenhohlraum 4b. Aus dieser Anordnung der Stützkörper 2 ist ersichtlich, wie sich die Stützkörper2 gegenseitig verkeilen, indem die Aussenkontur über die Öffnung 2c teilweise in den Innenhohlraum 3 eindringt, sodass sich die Stützkörper 2 gegenseitig verkeilen, und derart eine mechanisch zumindest zusammenhängende Stützstruktur ausbilden. Sobald der gesamte Knochenhohlraum 4b vollständig mit diesen Stützkörpern 2 gefüllt ist, kann die derart ausgebildete Stützstruktur die von Aussen auf den Knochen 4 einwirkende Last im Wesentlichen als Verbund tragen. Das in den Innenraum 4a eines Wirbelkörpers eingebrachte Füllmittel 1, umfassend eine Mehrzahl von beispielsweise 20 bis 50 Stützkörpern 2, bildet vorzugsweise eine eigenstabile Stützstruktur, indem die einzelnen Stützkörper 2 derart gegenseitig verkeilt sind, dass die dadurch gebildete Stützstruktur bei eine in Verlaufsrichtung der Wirbelsäule angreifenden Kraft zusammenhalten. Wäre der Innenraum 4a mit kugelförmigen Stützkörpern gefüllt, so würden diese bei einer in Verlaufsrichtung der Wirbelsäule angreifenden Kraft versuchen, in zur Kraft senkrechten Richtung zu entweichen, was eine übermässige Kraft auf die Aussenhülle des Wirbelkörpers erzeugt, oder sogar zur Beschädigung der Aussenhülle und zum Austreten der Stützkörper führen kann. Durch das gegenseitige Verkeilen der Stützkörper wird diese übermässige, auf die Aussenhülle wirkende Kraft erheblich reduziert. Die in Figur 9 dargestellte Anordnung der Stützkörper 2, beziehungsweise eine sehr ähnliche Anordnung, ergibt sich auch, wenn diese, wie in Figur 17 dargestellt, in den Wirbelkörper hineingepresst werden.

Figur 14 zeigt einen gebrochenen Knochen 4, wobei an der Bruchstelle ein Knochenteil fehlt. Diese Stelle ist mit einer Knochenplatte 10 abgedeckt, sodass sich ein Knochenhohlraum 4b ausbildet. Auch ein derartiger Knochenhohlraum 4b kann mit dem oben beschriebenen Füllmittel 1 umfassend Stützkörper 2 gefüllt werden.

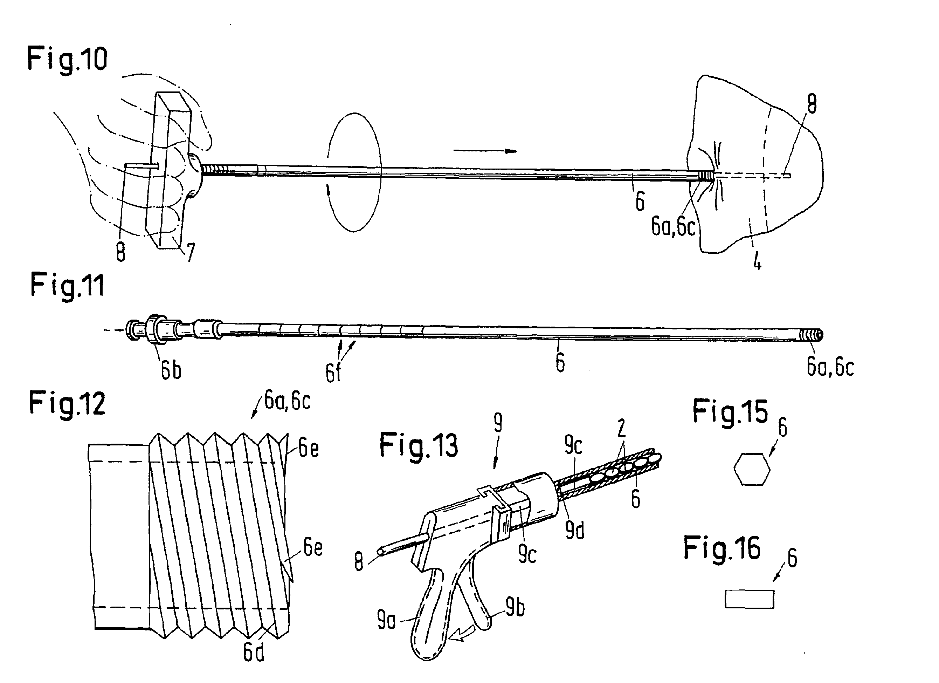

Figur 10 zeigt eine feste Kanüle 6 mit einer als Schneidgewinde 6c ausgestalteten Kanülenspitze 6a. Innerhalb der Kanüle 6 verläuft ein Kirschnerdraht 8. Vorerst wird der Kirschnerdraht 8 in den Knochen 4 gestossen, um die Verlaufsrichtung der Kanüle 6 zu bestimmen. Danach wird die Spitze 6a der Kanüle 6 unter Verwendung des Handgriffes 7 mit einer rotierenden Bewegung in den Knochen 4 geschraubt, sodass die Spitze 6a und somit die Kanüle 6 fest im Knochen 4 verankert ist. Danach wird der Handgriff 7 abgenommen. Figur 11 zeigt die Kanüle 6 mit entferntem Handgriff 7. Die Kanüle 6 umfasst ein Anschlussteil 6b für den Handgriff 7 beziehungsweise für eine Pressvorrichtung 9. Figur 12 zeigt ein Ausführungsbeispiel einer Spitze 6a der Kanüle 6 im Detail. Die Spitze 6a weist vorstehende Schneidkörper 6e und ein Aussengewinde 6d auf. Figur 13 zeigt eine Pressvorrichtung 9, welche über das Anschlussteil 6b mit der Kanüle 6 verbunden ist. Vor dem Verbinden wurde die Kanüle 6 mit Stützkörpern 2 gefüllt. Die Pressvorrichtung 9 umfasst einen Handgriff 9a sowie einen Betätigungsgriff 9b, welcher derart auf einen Stössel 9c wirkt, dass dieser sich bei jeder Betätigung ein Stück weit in die Kanüle 6 hineinbewegt, sodass der auf die Stützkörper 2 wirkende Stössel 9c die in der Kanüle 6 zuvorderst angeordneten Stützkörper 2 in den Knochen 4 presst. Die Stützkörper 2 fliessen derart in den Knochen 4 hinein, bilden darin auf Grund des Druckes zudem einen Knochenhohlraum 4b aus, und füllen diesen mit Stützkörpern 2, sodass innerhalb des Knochens 4 eine Stützstruktur ausgebildet wird. Die Lage des Stössels 9c kann mit Hilfe von an der Kanüle 6 angebrachten Markierungen 6f abgelesen werden, woraus abgeleitet werden kann wie viele Stützkörper 2 in den Knochen 4 gepresst wurden. Die Stützkörper 2 könnten auch in einer zusätzlichen Kanüle angeordnet sein, welche in die Kanüle 6 eingeführt wird, bevor die Pressvorrichtung 9 mit der Kanüle 6 verbunden wird. Somit wäre innerhalb der Kanüle 6 eine zweite Kanüle angeordnet, welche die Stützkörper 2 enthält. Derart könnte die Kanüle 6 sehr einfach mit den Stützkörpern 2 beschickt werden. Die die Stützkörper 2 enthaltende Kanüle muss einen derart der Grösse der Stützkörper 2 angepassten Innendurchmesser aufweisen, dass die Stützkörper 2 nur nacheinander folgend angeordnet und darin verschiebbar sind, um die Stützkörper 2 nacheinanderfolgend dem Knocheninnenraum 4a zuzuführen. Nach dem Füllen des Knocheninnenraumes 4a wird die Pressvorrichtung 9 und die Kanüle 6 entfernt und das Loch im Knochen, wie in Figur 4 dargestellt, mit einem Pfropfen 4c verschlossen.

Die Kanüle 6 kann nicht nur, wie in den Figuren 10 bis 13 dargestellt, einen runden Innenquerschnitt aufweisen, sondern auch andere Querschnittsformen, beispielsweise wie in Figur 15 dargestellt einen sechseckigen oder wie in Figur 16 dargestellt einen rechteckigen Innenquerschnitt, welche beispielsweise zum Einführen des in Figur 6a dargestellten Stützkörpers 2 geeignet sind. Die Kanüle 6 kann auch weitere, der jeweiligen Kontur des verwendeten Stützkörpers 2 angepasste Querschnittformen aufweisen.

Die Spitze 6a der Kanüle 6 kann auch ohne Befestigungsmittel 6c ausgestaltet sein. Insbesondere falls bereits vorgängig ein Knochenhohlraum 4b gebildet wurde, können die Stützkörper 2, wie in Figur 2 dargestellt, auch mit einer Spritzen ähnlichen Vorrichtung 9 dem Knochenhohlraum 4b zugeführt werden. In einem vorteilhaften Verfahren wird nacheinander folgend jeweils eine Gruppe von Stützkörpern, beispielsweise 5 oder 10 Stützkörper, dem Knochenhohlraum 4b zugeführt, danach die Kanüle 6 entfernt, und mit Hilfe eines Stopfwerkzeuges die sich im Knochenhohlraum 4b befindlichen Stützkörper 2 zusätzlich hineingepresst und verdichtet, um danach wieder die Kanüle am Knochen 4 anzusetzen, und eine weitere Gruppe von Stützkörpern 2 in den Knochenhohlraum 4b einzuführen, und diese wiederum mit dem Stopfwerkzeug zu verdichten.

In einem möglichen Verfahrensschritt kann, nachdem die Stützkörper 2 in den Knocheninnenraum 4a eingeführt wurden, eine osteoinduktive und/oder osteokonduktive Substanz zugeführt, beispielsweise als Flüssigkeit oder als Fluid, um die noch vorhandenen Hohlräume im Knocheninnenraum 4a mit dieser Substanz zu füllen. Das Füllmittel 1 könnte auch gemeinsam mit einem Fluid dem Knocheninnenraum 4a zugeführt werden, indem beispielsweise die Stützkörper 2 gemeinsam mit einem Fluid, insbesondere gemischt, in der Pressvorrichtung 9 zum Einführen in den Knocheninnenraum 4a bereitstehen.

Figur 17 zeigt einen Längsschnitt durch einen Wirbelkörper 4, in dessen Knocheninnenraum 4a eine Kanüle 6 eingeführt ist. Die Stützkörper 2 sind wie in Figur 6a dargestellt ausgestaltet, und weisen in Richtung der Achse A beabstandet angeordnete Stossstellen 2g auf, welche in Richtung der Achse A aneinander liegen und sich gegenseitig berühren. Die Stützkörper 2 werden mittel eines in Richtung B bewegten Stössels 9c, auf welchen eine Kraft F wirkt, in den Knocheninnenraum 4a geschoben beziehungsweise gedrückt, wobei sich die Stützkörper 2 im Knocheninnenraum 4a willkürlich anordnen, und im dargestellten Ausführungsbeispiel zudem einen Knochenhohlraum 4b ausbilden. Die sich in der Kanüle 6 befindlichen Stützkörper 2 sind nacheinander folgend, und ansonst bezüglich ihrer Lage identisch angeordnet, falls eine Kanüle 6 gemäss Figur 15 verwendet wird. Wird eine Kanüle 6 mit kreisförmigem Querschnitt verwendet, so sind die Stützkörper 2 auch nacheinander folgend angeordnet, wobei diese bezüglich ihrer gegenseitigen Lage auch um die Achse A verdreht angeordnet sein könnten. Da die Stützkörper 2 innerhalb der Kanüle 6 definiert in Verlaufsrichtung der Achse A beziehungsweise in Bewegungsrichtung B angeordnet sind, können die Stützkörper 2 zuverlässig und unter geringem Widerstand zum Knocheninnenraum 4a hin verschoben werden. Die Stützkörper 2 können radial zur Achse A auf unterschiedlichste Weise strukturiert sein, und beispielsweise Kanten 2d, Kerben 2h, oder auch vorstehende Spitzen, oder Ausnehmungen aufweisen, ohne dass diese Strukturen das Einführen der Stützkörper durch die Kanüle 6 behindern. Die Kräfte werden zuverlässig zwischen den Stossstellen 2g der Stützkörper 2 übertragen, und es findet innerhalb der Kanüle 6 kein gegenseitiges Verkeilen der Stützkörper statt, sodass diese zuverlässig bis in den Knocheninnenraum 4a geschoben werden. Innerhalb des Knocheninnenraumes 4a ordnen sich die Stützkörper 2 willkürlich an, sodass sich diese in ihrer Bewegung gegenseitig behindern, und sich gegenseitig verkeilen und blockieren.

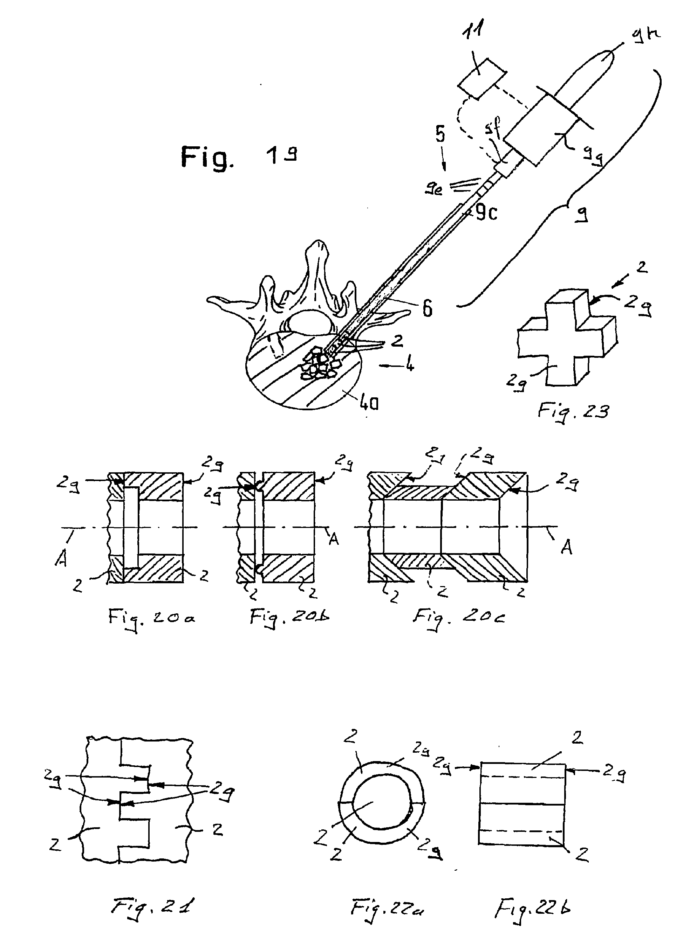

Figur 19 zeigt in einem Querschnitt eines Wirbelkörpers 4 eine Vielzahl von etwa 20 eingeführten Stützkörpern 2, welche innerhalb des Knocheninnenraumes 4a zufällig und wolkenförmig verteilt angeordnet sind. Um den Knocheninnenraum 4a gleichmässig zu füllen wird die Pressvorrichtung 9 nach dem ersten Einführen der Stützkörper 2 vorzugsweise an die mit 4c bezeichnete Stelle angesetzt, um an dieser Stelle ebenfalls Stützkörper 2 einzuführen.

Der Stössel 9c kann Markierungen 9e aufweisen, um die Eindringtiefe des Stössels 9c zu überwachen. Die Länge des Stössels 9c kann derart gewählt sein, dass dessen vorderer Teilabschnitt bis in den Knocheninnenraum 4a einführbar ist, beispielsweise bis zu einem Zentimeter. Die Spitze des Stössels9c kann, wie in den Figuren 18a und 18b dargestellt, flach oder abgerundet ausgestaltet sein. Derartige Stössel werden auch als Verdichtungsstössel bezeichnet. Vorzugsweise umfasst die Zuführvorrichtung 5 eine Mehrzahl unterschiedlich ausgestalteter Stössel 9c. Beispielsweise könnte der Stössel 9c eine wie in Figur 18b dargestellt, schräg verlaufende Spitze aufweisen. Diese kann dazu dienen, bereits innerhalb des Wirbelkörpers 4 angeordnete Stützkörper 2 auszurichten oder zu verschieben. Ein derartiger Stössel 9c wird auch als Positionierungsstössel bezeichnet. Die Lage der Stützkörper 2 im Wirbelkörper 4 kann mit Hilfe eines Röntenbildes sichtbar gemacht werden, sodass ein Arzt die Stützkörper 2 mit entsprechend ausgestalteten Werkzeugen wie Stösseln, Haken, oder Zangen verändern oder korrigieren kann. Figur 18d zeigt einen Stössel 9c mit einer ansteuerbar beweglich angeordneten Spitze. Figur 18e zeigt einen Stössel 9c mit einem vorstehenden Führungsteil, welches beispielsweise in den Innenhohlraum 3 eines Sützkörpers 2 eingreifen kann.

Die in Figur 19 dargestellte Pressvorrichtung 9 umfasst eine Kraftmessvorrichtung 9f, mit welcher die in Bewegungsrichtung B erzeugte Kraft gemessen und vorzugsweise auch direkt angezeigt wird. Die Kraftmessvorrichtung 9f könnte beispielsweise eine Feder sowie eine Anzeige umfassen, damit der Arzt die auf die Stützkörper 2 ausgeübte Kraft direkt messen kann. Die Kraftmessvorrichtung 9f könnte beispielsweise auch als elektronische Vorrichtung umfassend beispielsweise einen Piezokristall sowie eine Anzeige oder eine akustische Ausgabe ausgestaltet sein, wobei das gemessene Signal auch einer Überwachungsvorrichtung 11 zugeführt werden kann. Bevorzugt wird die maximal auf die Stützkörper 2 ausgeübte Kraft begrenzt. Die Kraftmessung ergibt einen Hinweis auf den Füllzustand beziehungsweise die Verkeilung der Stützkörper. Die Pressvorrichtung 9 umfasst vorzugsweise einen Handgriff 9h.

Die Pressvorrichtung 9 umfasst auch einen Antrieb 9g, um ein vibrierende Kraft auf die Stützkörper 2 zu bewirken. In einem vorteilhaften Verfahrensschritt werden eine Mehrzahl von Stützkörpern 2 dem Knocheninnenraum 4a zugeführt, und danach eine vibrierende Kraft auf die Stützkörper 2 ausgeübt, um die sich im Knocheninnenraum 4a befindlichen Stützkörper 2 zu verdichten, und um somit eine hohe Packungsdichte zu erzeugen, und um dadurch eine hohe Anzahl von Stützkörpern 2 dem Knocheninnenraum 4a zuzuführen. Die Vibrationsfrequenz liegt vorzugsweise im Bereich zwischen 1 und 15000 Hz, insbesondere zwischen 5 und 50 Hz.

In einer vorteilhaften Ausgestaltung erzeugt die Antriebsvorrichtung 9g eine elastische Stosswelle, welche in den Stössel 9c übertragen wird, was zur Folge hat, dass die Länge des Stössels 9c variiert wird, beispielsweise um +/- 2 mm, bei einer Frequenz von vorzugsweise zwischen 1 und 50 Hz.

Die Antriebsvorrichtung 9g könnte auch mit der Überwachungsvorrichtung 11 verbunden sein, und die maximale Kraft und/oder die Frequenz und/oder der Hub überwacht werden.

Die Figuren 20a bis 20c zeigen unterschiedlich ausgestaltete Stützkörper 2 mit runder oder beispielsweise sechseckiger Aussenkontur in einem Längsschnitt, entlang der Achse A. In Figur 20 a sind die Stossstellen 2g als Stossflächen ausgestaltet, wobei die links angeordnete Stossstelle 2g, im vergleich zur rechts angeordeten Stossstelle 2g, eine reduzierte Auflagefläche aufweist. Der in Figur 20b dargestellte Stützkörper 2 weist links eine vollständig in Umfangsrichtung verlaufende, kreisförmige Stossstelle 2g auf. Es könnte in Umfangsrichtung vereilt auch eine Mehrzahl von einzelnen, beispielsweise halbkugelförmig vorstehenden Elementen angeordnet sein, wobei jedes Element eine Stossstelle 2g bildet. Die in Figur 20c dargestellten Stützkörper 2 weisen Stossstellen 2g auf, welche als kegelstumpfförmige Flächen ausgestaltet sind. Ein Vorteil dieser Ausgestaltung ist; dass sich die Stützkörper 2 beim Schieben in Richtung der Achse A gegenseitig selbst zentrieren. Die Stützkörper 2 können, wie in Figur 20c dargestellt, unterschiedliche Aussendurchmesser aufweisen.

Figur 21 zeigt in einer Aussenansicht eine Abwicklung der Stossstellen 2g zwischen den beiden Stützkörpern 2. Diese flächig ausgestalteten Stossstellen 2g greifen gegenseitig ineinander, was ein gegenseitiges Verdrehen benachbarter Stützkörper 2 während dem Einschieben verhindert. Die Stossstellen 2g könnten formschlüssig ausgestaltet sein.

Figur 22a zeigt in einer Seitensicht drei Stützkörper 2, zwei U-förmige Stützkörper 2, welche einen zylinderförmigen Stützkörper 2 umschliessen. Diese drei Stützkörper 2 werden derart in der Kanüle 6 angeordnet, dass sie, wie in einer Seitenansicht in Figur 22b dargestellt, über die Stossstellen 2g in Richtung der Achse A dem Knocheninnenraum 4a zugeführt werden, wobei sich die drei Stützkörper 2 im Knocheninnenraum 4a zufällig anordnen, da diese gegenseitig nicht verbunden sind.

Die Oberfläche des sich innerhalb der beiden U-förmigen Stützkörper 2 befindlichen Stützkörpers 2 kann beliebig ausgestaltet sein, und beispielsweise auch Kanten oder Zacken aufweisen, auch an dessen Stirnflächen. Die in den Figuren 20a-20c sowei 22a und 22b dargestellten Stützkörper 2 können radial zur Achse A zum Beispiel kreisförmig, oder mehreckig ausgestaltet sein, oder auch Spitzen, Kanten, Ausnehmungen oder Durchbrechungen aufweisen.

Die Stirnfläche des Stössels 9c kann in einer Vielzahl von Formen ausgestalte sein, und beispielsweise auch die in den Figuren 20a bis 20c dargestellten Stirnseiten aufweisen.

Figur 23 zeigt einen weiteren Stützkörper 2 mit Stossflächen 2g. Die Arme könnten in einer Vielzahl unterschiedlicher Formen ausgestaltet sein, wobei zu gewährleisten ist, dass auf der Vorderseite sowie der Rückseite je eine Stossfläche 2g oder eine Stossstelle 2g vorhanden ist.

Auch die in den Figuren 8a bis 8d dargestellten Stützkörper 2 könnten in Richtung einer Achse A beabstandete Stossstellen 2g aufweisen. Beispielsweise könnte der in Figur 8b dargestellte Stützkörper 2 an der obersten und untersten Spitze eine wie in Figur 20c dargestellte, selbst zentrierende Stossstelle 2g aufweisen, sodass eine Mehrzahl derartiger, in einer Kanüle 6 hintereinander angeordneter Stützkörper 2, die sich an den Stossstellen 2g gegenseitig berühren, in Richtung der Achse A verschiebbar und dem Knocheninnenraum 4a somit zuführbar sind.

## Patentansprüche

1. Trockenfliessfähiges Füllmittel (1) zum Ausbilden einer Stützstruktur in einem Knocheninnemraum (4a), umfassend eine Mehrzahl biokompatibler Stützkörper (2), welche unter den üblicherweise im Knocheninneraum (4a) auftretenden physiologischen Lasten resistent gegen Verformung oder Bruch sind, wobei die Stützkörper (2) eine Grösse zwischen 2 mm und 10 mm ausweisen, und wobei die Stützkörper (2) eine Achse (A) aufweisen, sowie zwei gegenüberliegende, in Richtung der Achse (A) beabstandete Stossstellen (2g), **dadurch gekennzeichnet, dass** die Stossstellen (2g) senkrecht zur Achse (A) verlaufen, dass der Stützkörper (2) zwischen den Stossstellen (2g) eine bezüglich der Achse (A) in Umfangsrichtung verlaufende, eckige Aussenkontur mit ebenen Flächen (2f) aufweist, und dass die ebenen Flächen (2f) durch parallel zur Achse (A) verlaufende Kanten (2d) begrenzt sind.

2. Faltmittel nach Anspruch 1, **dadurch gekennzeichnet, dass** der Stützkörper (2) eine 3 bis 10-eckige Aussenkontur aufweist.

3. Füllmittel nach Anspruch 2, **dadurch gekennzeichnet, dass** der Stützkörper (2) eine 4-, 5- oder 6-eckige Aussenkontur aufweist.

4. Füllmittel nach einem der Ansprüche 1 bis 3, **dadurch gekennzeichnet, dass** die Stossstelle (2g) eine Ausnehmung (2c) aufweist.

5. Füllmittel nach Anspruch 4, **dadurch gekennzeichnet, dass** die Stützkörper (2) eine derart geformte Aussenkontur aufweisen, dass die Aussenkontur eines in den Knocheninnenraum (4a) eingefüllten Stützkörpers (2) in die Ausnehmung (2c) eines benachbart angeordneten Stützkörpers (2) hineinragen kann.

6. Füllmittel nach Anspruch 5, **dadurch gekennzeichnet dass** die Aussenkontur sowie die Ansnehmung (2c) der Stützkörper (2) derart gegenseitig angepasst geformt ist, dass zwischen den in Ansnehmungen (2c) hineinragende Aussenkonturen und der Ausnehmung (2c) ein gegenseitiges Verkeilen auftritt.

7. Füllmittel nach Anspruch 6, **dadurch gekennzeichnet, dass** die Verkeilung derart erfolgt, dass sich innerhalb des Knochenhehlraums (4a) eine zusammenhängende, eigenstabile Stützstruktur bildet.

8. Füllmittel nach einem der Anspruch 4 bis 7, **dadurch gekennzeichnet, dass** die Ausnehmung (2c) konzentrisch zur Achse (A) verläuft.

9. Füllmittel nach einem der Ansprüche 4 bis 8, **Dadurch gekennzeichnet, dass** der Stützkörper (2) einen offenen Innenhohlraum (3) aufweiset, welcher in die gegenüberliegenden Ausnehmungen (2c) mündet.

10. Füllmittel nach Anspruch 9, **dadurch gekennzeichnet, dass** der Innenhohlraum (3) zylinderförmig ausgestaltet ist; und in Richtung der Achse (A) verläuft.

11. Füllmittel nach Anspruch 9 oder 10, **Dadurch gekennzeichnet, dass** jeder Stützkörper (2) ein Gesamtvolumen beansprucht, welches das Volumen das Materials des Stützkörpers (2) sowie dessen Innenhohlraum (3) umfasst, wobei das Volumen des Innenhohlraumes (3) mehr als 30% des Gesamtvolumens beträgt.

12. Füllmittel nach Anspruch 11, **dadurch gekennzeichnet, dass** das Volumen des Innenhohlraumes (3) mehr als 50% des Gesamtvolumens beträgt.

13. Füllmittel nach einem der vorhergehenden Ansprüche, **Dadurch gekennzeichnet, dass** alle Stützkörper (2) bezüglich Grösse und Form identisch ausgestaltet sind.

14. Füllmittel nach einem der Ansprüche 4 bis 13, **dadurch gekennzeichnet, dass** die Ausnehmung (2c) Blockierstellen (2h) wie Kerben aufweisen.

15. Füllmittel nach einem der vorhergehenden Ansprüche, **dadurch gekennzeichnet, dass** die Kanten (2d) Blockierstellen (2h) wie Kerben ausweisen.

16. Füllmittel nach einem der vorhergehenden Ansprüche, **dadurch gekennzeichnet, dass** der Stützkörper (2) einen Aussendurchmesser (D) und orthogonal dazu, in Richtung der Achse (A), eine Höhe (H) aufweist, und dass der Aussendurchmesser (D) zumindest das 1,5-fache der Höhe (H) beträgt.

17. Füllmittel nach Anspruch 1, **dadurch gekennzeichnet, dass** der Stützkörper (2) eine einer Schraubenmutter ähnliche Form aufweist, mit zwei senkrecht zur Achse (A) verlaufenden Stossstellen (2g), und mit einer mehreckigen Aussenkontur mit parallel zur Achse (A) verlaufenden Kanten.

18. Füllmittel nach Ansprüche 17, **dadurch gekennzeichnet dass** der Stützkörper (2) einen in Richtung der Achse (A) verlaufenden, im wesentlichen zylinderförmigen Innenhohlraum (3) aufweist, welcher beidseitig in die Stossstellen (2g) mündet.

19. Füllmittel nach einem der vorhergehenden Ansprüche, **dadurch gekennzeichnet**, daas die Stützkörper (2) bestehen aus:
- keramischen Werkstoffen, insbesondere Calciumphosphate / Hydroxylapathie, Aluminiumoxide, Zirkoniumoxide, ATZ Keramik (Alu-Zirkonium-Oxid), bioaktive Gläser, Glaskeramiken. Porzellan oder einer Kombination davon, oder
- metallischen Werkstoffen, insbesondere. Titan, Tantal, rostfreier Stahl, Stahltegierungen wie Kobalt-Chrom Legierumg, Titanlegierungen wie Titan-Nickel Legierung oder Titan-Aluminium-Niobium/Vanadium Legierung, oder einer Kombination davon, oder
- Polymeren, insbesondere Polymethylmethacryl (PMMA), Polyetheretherketon (PEEK) Polyethylen (PE). Polyethylenterephthalat (PET), oder einer Kombination davon, oder
- biodegradablen Polymeren wie Polylactate.

20. Füllmittel nach einem der Ansprüche 4 bis 19, **dadurch gekennzeichnet, dass** die Ausnehmung (2c) oder der Innenhohlraum (3) mit zumindest eine osteoinduktive und/oder osteokonduktiven Substanz gefüllt ist, insbesondere knochenwachstumsförderndes Protein, Kalziumzulfat, oder eine Kombination davon.

21. Füllmittel nach einem der vorhergehenden Ansprüche, **dadurch gekennzeichnet, dass** der Stützkörper (2) aus mehreren Teilkörpern besteht.

22. Zuführvorrichtung (5) zum Einführen eines trockenfliessfähigen Füllmittels (1) nach einem der vorhergehenden Ansprüche in einem Knocheninnenraum (4a), wobei die Zuführvorrichtung (5) eine Kanüle (6) umfasst, mit einem derart der Grösse der Stützkörper (2) angepassten Innenquerschnitt, dass die Stützkörper (2) innerhalb der Kanüle (6) nacheinander folgend und in Richtung der Achse (A) ausgerichtet dem Knocheninnenraum (4a) zuführbar sind, und wobei die Zuführvorrichtung (5) eine Pressvorrichtung (9) umfasst, mit einem in der Kanüle (6) verschiebbaren Stössel (9c), mit welchem auf die sich in der Kanüle (6) befindlichen Stürzkörper (2) eine Kraft in Förderrichtung ausübbar ist, um die Stützkörper (2) unter Druck dem Knocheninnenraum (4a) zuzuführen, wobei die Pressvorrichtung (9) eine Antriebsvorrichtung (9g) umfasst, welche den Stössel (9c) in Förderrichtung antreibt, und wobei die Antriebsvorrichtung (9g) eine vibrierende Kraft auf den Stössel (9c) ausübt.

23. Zuführvorrichtung nach Anspruch 22, wobei die Pressvorrichtung (9) eine Kraftmessvorrichtung (9d) umfasst, zum Messen der in Förderrichtung auf die Stützkörper (2) bewirkten Stosskraft.

24. Zuführvorrichtung nach einem der Ansprüche 22 bis 23, wobei der Stössel (9c) eine derartige Länge aufweist, dass dessen vorderer Teilabschnitt bis in den Knocheninnenraum (4a) einführbar ist.

25. Zuführvorrichtung nach Anspruch 24, umfassend einen Stössel (9c) mit einer derart ausgeformten oder einer derart beweglichen Spitze, dass diese an einem sich innerhalb des Knocheninnenraumes (4a) befindlichern Füllmittel (1) angreifen, und dessen Lage, insbesondere quer zur Förderrichtung, verändern kann.

26. Zuführvorrichtung nach Anspruch 22, **dadurch gekennzeichnet, dass** die Antriebsvorrichtung (9g) eine elastische Stosswelle erzeugt, welche in den Stössel(9c) übertragbar ist.

27. System zum Fixieren und wieder Erweitern eines teilweise eingebrochenen Wirbelkörpers, umfassend
- eine Mehrzahl von Füllmittel gemäss den Ansprüchen 1 bis 21, und
- eine Zuführvorrichtung (5) umfassend eine Kanüle (6) mit einem derart der Grösse der Stützkörper (2) angepassten Innenquerschnitt, dass die Stützkörper (2) innerhalb der Kanüle (6) nacheinander folgend, in gleich ausgerichteter Lage und gegenseitig an den Stossstellen (2g) anliegend dem Knocheninnenraum (4a) in Richtung der Achse (A) zuführbar sind, sowie umfassend einen in der Kanüle (6) verschiebbaren Stössel (9c), mit welchem auf den hintersten, der sich in der Kanüle (6) befindlichen Stützkörpern (2) eine Kraft in Förderrichtung (A) ausübbar ist.

## Claims

1. A dry flowable filler means (1) for the formation of a support structure in an internal bone space (4a), comprising a plurality of biocompatible support bodies (2) which are resistant to deformation or fracture under the typically-arising physiological loads within the internal bone space (4a), with the support bodies (2) having a size between 2 mm and 10 mm and wherein the support bodies (2) have an axis (A) and also two abutment locations (2g) each facing in opposite directions and spaced apart from each other along the direction of the axis (A), **characterized in that**
the abutment locations (2g) extend perpendicular to the axis (A), **in that**, in between the abutment locations (2g), the support bodies (2) have, in circumferential direction of the axis (A), a polyhedral outer contour with planar surfaces (2f), and that the planar surfaces (2f) are delimited by edges (2d) extending parallel to the axis (A).

2. Filler means in accordance with claim 1, **characterized in that** the support bodies (2) have a 3-cornered to 10-cornered outer contour.

3. Filler means in accordance with claim 2, **characterized in that** the support bodies (2) have a 4-cornered, 5-corndered or 6-cornered outer contour.

4. Filler means in accordance with one of claims 1 to 3, **characterized in that** the abutment location (2g) has a recess (2c).

5. Filler means in accordance with claim 4, **characterized in that** the support bodies (2) have an outer contour shaped such that the outer contour of a support body (2) filled into the internal bone space (4a) can project into the recess (2c) of an adjacently disposed support body (2).

6. Filler means in accordance with claim 5, **characterized in that** the outer contour and also the recess (2c) of the support bodies (2) are shaped in a mutually fitting manner such that mutual wedging occurs between the outer conture projecting into the recesses (2c) and the recess (2c).

7. Filler means in accordance with claim 6, **characterized in that** the wedging takes place in such a way that a cohesive self-stable support structure is formed within the internal bone space (4a).

8. Filler means in accordance with one of claims 4 to 7, **characterized in that** the recess (2c) extends concentrically to the axis (A).

9. Filler means in accordance with one of the claims 4 to 8, **characterized in that** the support bodies (2) have an open inner hollow cavity (3) which opens into the oppositely disposed recesses (2c).

10. Filler means in accordance with claim 9, **characterized in that** the inner hollow cavity (3) is of cylindrical shape and extends in the direction of the axis (A).

11. Filler means in accordance with claim 9 or 10, **characterized in that** each support body (2) takes up a total volume which includes the volume of a material of the support body (2) and also its inner hollow cavity (3), with the volume of the inner hollow cavity (3) amounting to more than 30 % of the total volume.

12. Filler means in accordance with claim 11, **characterized in that** the volume of the inner hollow cavity (3) amounts to more than 50 % of the total volume.

13. Filler means in accordance with any one of the preceding claims, **characterized in that** all support bodies (2) are identically designed with respect to size and shape.

14. Filler means in accordance with one of claims 4 to 13, **characterized in that** the recess (2c) has blocking points (2h) such as notches.

15. Filler means in accordance with any one of the preceding claims, **characterized in that** the edges (2d) have blocking points (2h) such as notches.

16. Filler means in accordance with any one of the preceding claims, **characterized in that** the support bodies (2) have an outer diameter (D) and orthogonal to it a height (H) in the direction of the axis (A) and **in that** the outer diameter (D) amounts to at least 1.5 times the height (H).

17. Filler means in accordance with claim 1, **characterized in that** the support body (2) has a form similar to a threaded nut having two abutment locations (2g) extending perpendicular to the axis (A) and with a multi-cornered outer counter with edges extending parallel to the axis (A).

18. Filler means in accordance with claim 17, **characterized in that** the support body (2) has an essentially cylindrical inner cavity (3) extending in the direction of the axis (A) which opens at both ends into the abutment location (2g).

19. Filler means in accordance with any one of the preceding claims, **characterized in that** the support bodies (2) consists of
- ceramic materials, in particular calcium phosphate/hydroxylapatite, aluminum oxide, zirconium oxide, ATZ ceramic (aluminum zirconium oxide), bioactive glasses, glass ceramic materials, porcelain or a combination thereof or
- metallic materials in particular titanium, tantalum, stainless steel, steel alloys such as cobalt chrome alloy, titanium alloys such as titanium nickel alloy or titanium aluminum niobium/vanadium alloy or a combination thereof, or
- polymers, in particular polymethyl methacrylate (PMMA), polyetheretherketone (PEEK), polyethylene (PE), polyethylene terephthalate (PET) or a combination thereof, or
- biodegradable polymers such as polylactate.

20. Filler means in accordance with one of claims 4 to 19, **characterized in that** the recess (2c) or the interior cavity (3) is filled with at least one osteo-inductive and/or osteo-conductive substance, in particular bone growth promoting protein, calcium sulphate or a combination thereof.

21. Filler means in accordance with any one of the preceding claims, **characterized in that** the support bodies (2) consists of a plurality of partial bodies.

22. A feeding device (5) for the supplying of a filler means (1) according to one of the preceding claims, the filler means (1) capable of dry flowing into an internal bone space (4a), wherein the supply device (5) includes a cannula (6) with a cross-section matched to that of the size of the support bodies (2) in such a way that the support bodies (2) can be supplied following one another within the cannula (6) aligned in the direction of the axis (A) to the internal bone space (4a) and with the feeding device (5) including a pressing device (9) having a plunger (9c) displaceable in the cannula (6) with which a force can be exerted in the feeding direction on the support bodies (2) located within the cannula (6) in order to supply the support bodies (2) to the internal bone space (4a) under pressure, wherein the pressing device (9) includes a drive device (9g) which drives the plunger (9c) in the feeding direction, and wherein the driving apparatus (9g) exerts a vibrating force on the plunger (9c).

23. Feeding device in accordance with claim 22, wherein the pressing apparatus (9) includes a force measuring device (9d) for the measuring of the thrust force acting on the support bodies (2) in the feeding direction.

24. Feeding device in accordance with one of the claims 22 to 23, wherein the plunger (9c) has a length such that its front part section can be introduced up to and into the internal bone space (4a).

25. Feeding device in accordance with claim 24 including a plunger (9c) having a tip formed on it or movable on it such that it can act on a filler means (1) located within the internal cavity (4a) of the bone and change its position, in particular transverse to the feeding direction.

26. Feeding device in accordance with claim 22, **characterized in that** the driving apparatus (9g) produces an elastic shock wave which is transferred into the plunger (9c).

27. A system for the fixing and further enlargement of a partly fractured vertebral body including
- a plurality of filler means in accordance with one of claims 1 to 21, and
- a feeding device (5) including a cannula (6) having an internal cross-section matched to the size of the support bodies (2) such that the support bodies (2) can be fed within the cannula (6) following one another in the same aligned position and contacting one another mutually at the abutment locations (2g) to the internal bone space (4a) in the direction of the axis (A) and also comprising a plunger (9c) displaceable in the cannula (6) with which a force can be exerted in the feeding direction (A) onto the rearmost of the support bodies (2) located in the cannula (6).

## Revendications

1. Moyen de remplissage pouvant s'écouler à sec (1), destiné à former une structure de soutien dans une cavité osseuse (4a), comprenant une pluralité de corps de soutien biocompatibles (2) qui sont résistants à la déformation ou à la rupture sous l'effet des charges physiologiques habituellement présentes dans la cavité osseuse (4a), les corps de soutien (2) présentant une dimension comprise entre 2 mm et 10 mm, et les corps de soutien (2) comportant un axe (A) ainsi que deux abouts (2g) opposés et espacés dans la direction de l'axe (A), **caractérisé en ce que** les abouts (2g) s'étendent perpendiculairement à l'axe (A), que le corps de soutien (2) présente entre les abouts (2g) un contour extérieur polygonal qui comporte des surfaces planes (2f) et qui s'étend en direction circonférentielle par rapport à l'axe (A), et que les surfaces planes (2f) sont limitées par des arêtes (2d) s'étendant parallèlement à l'axe (A).

2. Moyen de remplissage selon la revendication 1, **caractérisé en ce que** le corps de soutien (2) présente un contour extérieur ayant 3 à 10 angles.

3. Moyen de remplissage selon la revendication 2, **caractérisé en ce que** le corps de soutien (2) présente un contour extérieur ayant 4, 5 ou 6 angles.

4. Moyen de remplissage selon une des revendications 1 à 3, **caractérisé en ce que** l'about (2g) présente un évidement (2c).

5. Moyen de remplissage selon la revendication 4, **caractérisé en ce que** les corps de soutien (2) présentent un contour extérieur formé de telle manière que le contour extérieur d'un corps de soutien (2) introduit dans la cavité osseuse (4a) puisse pénétrer dans l'évidement (2c) d'un corps de soutien (2) disposé au voisinage.

6. Moyen de remplissage selon la revendication 5, **caractérisé en ce que** le contour extérieur ainsi que l'évidement (2c) des corps de soutien (2) sont formés de manière à être mutuellement adaptés afin qu'il se produise un encastrement mutuel entre les contours extérieurs pénétrant dans les évidements (2c) et l'évidement (2c).

7. Moyen de remplissage selon la revendication 6, **caractérisé en ce que** l'encastrement s'effectue de telle manière qu'il se forme une structure de soutien auto-stable et cohérente à l'intérieur de la cavité osseuse (4a).

8. Moyen de remplissage selon une des revendications 4 à 7, **caractérisé en ce que** l'évidement (2c) s'étend concentriquement à l'axe (A).

9. Moyen de remplissage selon une des revendications 4 à 8, **caractérisé en ce que** le corps de soutien (2) présente une cavité intérieure (3) ouverte qui débouche dans les évidements (2c) opposés.

10. Moyen de remplissage selon la revendication 9, **caractérisé en ce que** la cavité intérieure (3) est de forme cylindrique et s'étend dans la direction de l'axe (A).

11. Moyen de remplissage selon la revendication 9 ou 10, **caractérisé en ce que** chaque corps de soutien (2) occupe un volume total qui comprend le volume du matériau du corps de soutien (2) ainsi que celui de sa cavité intérieure (3), le volume de la cavité intérieure (3) représentant plus de 30 % du volume total.

12. Moyen de remplissage selon la revendication 11, **caractérisé en ce que** le volume de la cavité intérieure (3) représente plus de 50 % du volume total.

13. Moyen de remplissage selon une des revendications précédentes, **caractérisé en ce que** tous les corps de soutien (2) sont identiques en ce qui concerne la taille et la forme.

14. Moyen de remplissage selon une des revendications 4 à 13, **caractérisé en ce que** l'évidement (2c) présente des points de blocage (2h) comme des encoches.

15. Moyen de remplissage selon une des revendications précédentes, **caractérisé en ce que** les arêtes (2d) présentent des points de blocage (2h) comme des encoches.

16. Moyen de remplissage selon une des revendications précédentes, **caractérisé en ce que** le corps de soutien (2) présente un diamètre extérieur (D) et, orthogonalement à celui-ci, dans la direction de l'axe (A), une hauteur (H), et que le diamètre extérieur (D) est au moins égal à 1,5 fois la hauteur (H).

17. Moyen de remplissage selon la revendication 1, **caractérisé en ce que** le corps de soutien (2) présente une forme analogue à un écrou, avec deux abouts (2g) s'étendant perpendiculairement à l'axe (A), et avec un contour extérieur polygonal comportant des arêtes s'étendant parallèlement à l'axe (A).

18. Moyen de remplissage selon la revendication 17, **caractérisé en ce que** le corps de soutien (2) présente une cavité intérieure (3) essentiellement cylindrique, s'étendant dans la direction de l'axe (A), qui débouche des deux côtés dans l'about (2g).

19. Moyen de remplissage selon une des revendications précédentes, **caractérisé en ce que** les corps de soutien (2) sont composés de :
- matériaux céramiques, en particulier de phosphates de calcium / d'hydroxylapatites, d'oxydes d'aluminium, d'oxydes de zirconium, de céramique ATZ (oxyde d'aluminium et de zirconium), de verres bioactifs, de vitrocéramiques, de porcelaine ou d'une combinaison de ceux-ci, ou de
- matériaux métalliques, en particulier de titane, tantale, acier inoxydable, alliages d'acier tels que alliage cobalt-chrome, alliages de titane tels que alliage titane-nickel ou alliage titan-aluminium-niobium/vanadium, ou d'une combinaison de ceux-ci, ou de
- polymères, en particulier de polyméthacrylate de méthyle (PMMA), polyetheretherketone (PEEK), polyéthylène (PE), polyéthylène téréphthalate (PET) ou d'une combinaison de ceux-ci, ou de
- polymères biodégradables tels que polylactate.

20. Moyen de remplissage selon une des revendications 4 à 19, **caractérisé en ce que** l'évidement (2c) ou la cavité intérieure (3) est rempli(e) d'au moins une substance ostéoinductrice et/ou ostéoconductrice, en particulier d'une protéine favorisant la croissance osseuse, de sulfate de calcium ou d'une combinaison de ceux-ci.

21. Moyen de remplissage selon une des revendications précédentes, **caractérisé en ce que** le corps de soutien (2) est composé de plusieurs sous-corps.

22. Dispositif d'introduction (5) pour introduire un moyen de remplissage pouvant s'écouler à sec (1) selon une des revendications précédentes dans une cavité osseuse (4a), le dispositif d'introduction (5) comprenant une canule (6) présentant une section intérieure adaptée à la taille des corps de soutien (2) de telle manière que les corps de soutien (2) puissent être introduits dans la cavité osseuse (4a) successivement et orientés dans la direction de l'axe (A) à l'intérieur de la canule (6), et le dispositif d'introduction (5) comprenant un dispositif de pressage (9) avec un poussoir (9c) mobile dans la canule (6), avec lequel une force peut être exercée en direction de transport sur les corps de soutien (2) se trouvant dans la canule (6) afin d'introduire les corps de soutien (2) sous pression dans la cavité osseuse (4a), le dispositif de pressage (9) comprenant un dispositif d'entraînement (9g) qui entraîne le poussoir (9c) dans la direction de transport, et le dispositif d'entraînement (9g) exerçant une force vibrante sur le poussoir (9c).

23. Dispositif d'introduction selon la revendication 22, dans lequel le dispositif de pressage (9) comprend un dispositif de mesure de force (9d) pour mesurer la force de propulsion exercée sur les corps de soutien (2) dans la direction de transport.

24. Dispositif d'introduction selon une des revendications 22 à 23, dans lequel le poussoir (9c) présente une longueur telle que sa partie avant puisse être introduite jusque dans la cavité osseuse (4a).

25. Dispositif d'introduction selon la revendication 24, comprenant un poussoir (9c) avec une pointe formée de telle manière ou mobile de telle manière que celle-ci puisse agir sur un moyen de remplissage (1) se trouvant à l'intérieur de la cavité osseuse (4a) et modifier sa position, en particulier transversalement à la direction de transport.

26. Dispositif d'introduction selon la revendication 22, **caractérisé en ce que** le dispositif d'entraînement (9g) produit une onde de choc élastique qui peut être transmise dans le poussoir (9c).

27. Système pour immobiliser et élargir de nouveau un corps de vertèbre partiellement effondré, comprenant
- une pluralité de moyens de remplissage selon les revendications 1 à 21, et
- un dispositif d'introduction (5) comprenant une canule (6) présentant une section intérieure adaptée à la taille des corps de soutien (2) de telle manière que les corps de soutien (2) puissent être introduits dans la cavité osseuse (4a) dans la direction de l'axe (A) successivement, avec la même orientation et en contact mutuel par leurs abouts (2g) à l'intérieur de la canule (6), ainsi que comprenant un poussoir (9c) mobile dans la canule (6) avec lequel peut être appliquée une force en direction de transport (A) sur les derniers corps de soutien (2) se trouvant dans la canule (6).
